(19)

Europäisches
Patentamt
European
Patent Office
Office européen
des brevets

(11) EP 2 685 399 A2

(12) EUROPEAN PATENT APPLICATION

(43) Date of publication:
15.01.2014 Bulletin 2014/03

(51) Int Cl.:
G06F 19/12 (2011.01)

(21) Application number: 13176048.0

(22) Date of filing: 11.07.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 13.07.2012 KR 20120076803

(71) Applicant: Samsung Electronics Co., Ltd
Gyeonggi-do 443-742 (KR)

(72) Inventors:
• Ahn, Tae-jin
Gyeonggi-do (KR)
• Mukherjee, Subhankar
560037 Bangalore (IN)

• Hong, Seok-jin
Gyeonggi-do (KR)
• Mallavarapu, Rama Srikanth
500076 Hyderabad (IN)
• Son, Dae-soon
Gyeonggi-do (KR)
• Lee, Chon-hee
Gyeonggi-do (KR)
• Bopardikar, Shyamsunder Ajit
560037 Bangalore (IN)

(74) Representative: Grünecker, Kinkeldey,
Stockmair & Schwanhäusser
Leopoldstrasse 4
80802 München (DE)

(54) **Method and apparatus for analyzing gene information for treatment selection**

(57) A method and apparatus for analyzing gene information for treatment selection are disclosed herein. In some embodiments, information about a gene network in which genes included in a genome of an individual are classified into a plurality of subgroups based on functional correlations between the genes is acquired, and subgroups corresponding to an action of at least one drug to be used are visualized.

FIG. 1

EP 2 685 399 A2

**Description**

BACKGROUND

1. Field

The present disclosure relates to methods and apparatuses for analyzing gene information, such as a genome of an individual, for treatment selection.

2. Description of the Related Art

The genome indicates the entire gene information of an organism. Various techniques of sequencing the genome of a certain individual, such as a DeoxyriboNucleic Acid (DNA) chip and Next Generation Sequencing (NGS) technique, a Next NGS (NNGS) technique, and so forth, have been developed. Analysis of gene information, such as a nucleic acid sequence and protein, is widely used to find a gene indicating a disease, such as diabetes or cancer, or perceive a correlation between a genetic variety and an individual expression characteristic. In particular, gene information collected from individuals is significant to find out a genetic characteristic of an individual associated with the progression of different symptoms or diseases. Thus, gene information, such as a nucleic acid sequence and protein of an individual, is core data for perceiving current and future disease-related information to prevent diseases or select an optimal therapy at an initial stage of a disease. Techniques of correctly analyzing gene information of individuals by using genome detecting devices, such as a DNA chip and a microarray for detecting Single Nucleotide Polymorphism (SNP), Copy Number Variation (CNV), and so forth, have been researched.

SUMMARY

Provided is a method and apparatus for analyzing gene information, such as the genome of an individual, for treatment selection, as well as a computer-readable recording medium storing a computer-readable program for executing the method.
According to an aspect of the present invention, a method of analyzing gene information for treatment selection is provided. The method comprising: acquiring  information about a gene network in which genes are classified into a plurality of subgroups based on functional correlations between the genes; extracting gene subgroups that include a gene targeted by at least one drug to be used in treatment from among the plurality of subgroups included in the gene network; and generating at least one index based on gene information included in the extracted subgroups to visualize the extracted subgroups, wherein one or more of the steps of the method are performed using a gene analyzing apparatus.
According to another aspect of the present invention, provided is an apparatus for analyzing gene information for treatment selection, the apparatus comprising: a data acquisition unit for acquiring information about a gene network in which genes are classified into a plurality of subgroups based on functional correlations between the genes; a subgroup extracting unit for extracting gene subgroups that include a gene targeted by at least one drug to be used in treatment from among the plurality of subgroups included in the gene network; and an index generating unit for generating at least one index based on gene information included in the extracted subgroups to visualize the extracted subgroups.

BRIEF DESCRIPTION OF THE DRAWINGS

These and/or other aspects will become apparent and more readily appreciated from the following description of the embodiments, taken in conjunction with the accompanying drawings in which:
FIG. 1 a block diagram of an apparatus for analyzing gene information for treatment selection;
FIG. 2 is a gene network;
FIG. 3A illustrates a table of a drug list that is input into the apparatus of FIG. 1 by a user;
FIG. 3B illustrates a table of subgroups extracted by a subgroup extracting unit;
FIG. 4 is a diagram showing an index of a genetic alteration level of an extracted subgroup, which is generated by an index generating unit;
FIG. 5A is a diagram for describing a process of estimating a distance in the index generating unit;
FIG. 5B is a diagram for describing a process of estimating a distance in the index generating unit;
FIG. 6 is a diagram showing a result processed by a visualization processor;
FIG. 7 is a diagram showing visualized results of a colon cancer sample of a responder and a colon cancer sample of a non-responder responding to Cetuximab; and
FIG. 8 is a flowchart illustrating a method of analyzing gene information for treatment decision according to an embodiment of the present invention.

DETAILED DESCRIPTION

Reference will now be made in detail to the following embodiments, examples of which are illustrated in the accompanying drawings.

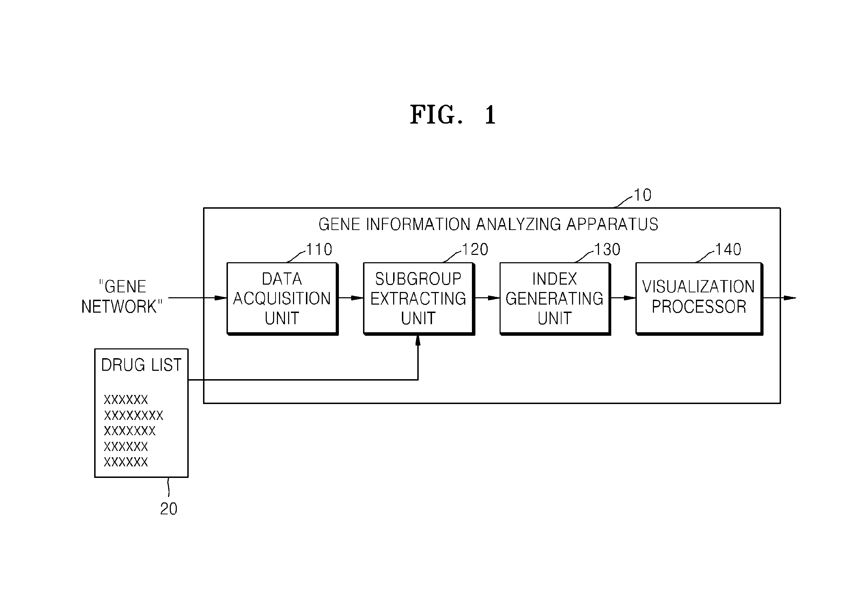

FIG. 1 is a block diagram of an apparatus 10 for analyzing gene information for treatment selection according to an embodiment of the present invention. Referring to FIG. 1, the apparatus 10 includes a data acquisition unit 110, a subgroup extracting unit 120, an index generating unit 130, and a visualization processor 140. For clarity reasons, only hardware components related to the current embodiment are described in FIG. 1. However, it will be understood by those of ordinary skill in the art that other general-use hardware components may be further included in the apparatus 10.

In particular, the apparatus 10 may be a processor. This processor may be implemented by an array having a plurality of logic gates or a combination of a microprocessor and a memory storing programs executable by the microprocessor. In addition, it will be understood by those of ordinary skill in the art that the apparatus 10 may also be implemented by another type of hardware.

The apparatus 10 may be used as a device for helping medical practitioners in patient diagnosis and treatment selection by visualizing gene information associated with a gene causing a disease, such as cancer or tumor, from among genome data of an individual in relation to drug use, such as an anticancer drug. In addition, information provided by the apparatus 10 may be used for research, such as the development of new medicines, diagnostic markers, and so forth.

In general, the genome of an individual indicates all gene information that the individual has, and recently, the complete genome of a human being and other organisms have been expressed following the development of sequencing technologies. Gene information included in the genome, such as a nucleic acid sequence, protein revelation, and so forth, is mandatory for finding out biological action mechanisms. Genome analysis is widely used to understand various biological phenomena, such as finding out the cause of a specific disease such as diabetes or cancer, a genetic variety, an individual expression characteristic, and so forth.

Recently, functional correlations between genes included in the genome have been gradually expressed in genome research, thereby making it possible to conduct analysis of a gene network among genes. This is because almost all physiological symptoms occurring in a certain living organism are due to interactions of several genes instead of a single gene.

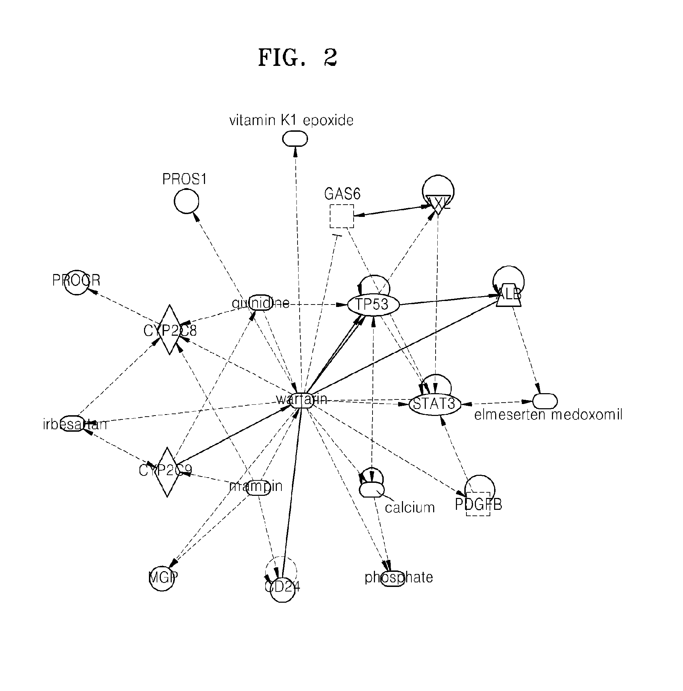

FIG. 2 illustrates an example gene network. FIG. 2 shows only a portion of the entire gene network to help in understanding the current embodiment. However, information about the remaining portion of the entire gene network may also be easily acquired by those of ordinary skill in the art.

Referring to FIG. 2, the gene network is represented as a network in which genes are connected to each other in a complicated manner. In particular, the gene network includes genes classified into a plurality of subgroups or subnets according to functional correlations between the genes. These subgroups or subnets are represented by nodes (e.g., genes or expression products, such as proteins) in the gene network shown in FIG. 2. For example, although not shown in the gene network of FIG. 2, when nodes corresponding to subgroups or subnets are marked using the symbols ALK, EPHA1, and JAK3, the nodes may indicate anaplastic lymphoma receptor tyrosine kinase, EPH receptor A1, and Janus kinase 3, respectively. Since the gene network described above is obvious to those of ordinary skill in the art, a detailed description thereof is omitted.

Even though information about a gene network is known, research on a method of analyzing the gene network in association with various medical treatments, such as drug therapy, have rarely been conducted. In particular, only techniques for measuring an alteration in a single gene or a set of genes of an individual cancer patient (an alteration in a cancer patient's cell against a normal cell) have been introduced for the case where a prescription of a certain type of anticancer drug is considered. However, techniques for measuring an alteration in a single gene or a set of genes of an individual cancer patient by taking correlations between these anticancer drugs into account have not been introduced for the case where a prescription of two or more types of anticancer drugs is considered.

When a prescription of two or more types of anticancer drugs is considered, it may be meaningless trying to determine the anticancer drugs by individually measuring an alteration in a gene set for each type of anticancer drug because it may be difficult to anticipate the full efficacy of two types of anticancer drugs when the two types of anticancer drugs have the same or similar mechanisms. Thus, when a customized therapy of two or more types of anticancer drugs is considered, it may be first determined whether a genetic alteration of a patient is related to the efficacy of each anticancer drug, and whether mechanisms of the two or more types of anticancer drugs are similar may be simultaneously measured. In other words, when several anticancer drugs are used, it may be measured whether several kinds of oncogenes are related to pathways of the several anticancer drugs, and if it is measured that several kinds of oncogenes are related to the pathways of the several anticancer drugs, correlations between the several anticancer drugs may be first perceived for the optimal joint use of anticancer drugs.

Unlike the existing apparatuses for analyzing gene information, the apparatus 10 may index correlations between several oncogenes related to several anticancer drugs in a gene network, numerically analyze the indexes, and provide the numerical result. That is, the apparatus 10 may numerically analyze and provide a relationship between several gene sets (subgroups or subnets) instead of numerically analyzing an alteration in a single gene or a single set of genes as in the existing apparatuses.

An operation and function of the apparatus 10 will now be described in more detail. Referring back to FIG. 1, the data acquisition unit 110 acquires information about a gene network in which genes included in an individual genome are classified into a plurality of subgroups (or subnets) according to functional correlations between the genes. The acquired information about the gene network may include information about an interconnection relationship between the genes included in the individual genome, information about the plurality of subgroups (or subnets) classified according to the functional correlations, and so forth. The acquired gene network may be acquired from a database (DB) already known in the art.

The subgroup extracting unit 120 extracts subgroups having a gene corresponding to an action of at least one drug to be used from among the plurality of subgroups included in the gene network acquired by the data acquisition unit 110.

A user of the apparatus 10, e.g., a medical practitioner, may input a list of anticancer drugs to be prescribed for a certain cancer patient by using the apparatus 10. Alternatively, the user of the apparatus 10 may input a list of drugs to research correlations between subgroups corresponding to certain drugs. Although not shown in FIG. 1, a general user interface device connected to the apparatus 10 may be used to input the list. The apparatus then maps the drugs to gene subgroups based on the known drug targets. By way of further illustration, the apparatus may identify the gene targets of each drug based on available information, and then identify and extract one or more gene subgroups to which the gene targets belong. A "gene target" or "gene targeted by a drug" refers to a gene that is directly or indirectly acted upon by a drug when administered to the body of a patient. A gene is acted upon by a drug if the expression of the gene or activity or concentration of the gene product (e.g., mRNA or protein) is increased or decreased in the presence of the drug as compared to the same expression, activity, or level in the absence of the drug.

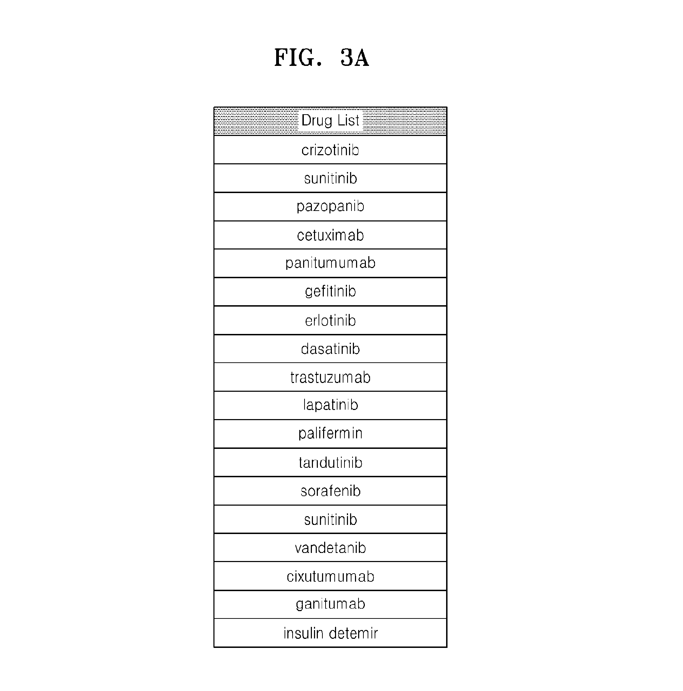

FIG. 3A illustrates a table of a drug list 20 inputted into the apparatus 10 of FIG. 1 by a user, according to an embodiment of the present invention. Referring to FIG. 3A, the names of 18 different anticancer drugs, such as crizotinib, sunitinib, pazopanib, cetuximab, panitumumab, gefitinib, erlotinib, dasatinib, trastuzumab, lapatinib, palifermin, tandutinib, sorafenib, sunitinib, vandetanib, cixutumumab, ganitumab, and insulin detemir, are listed in the drug list 20.

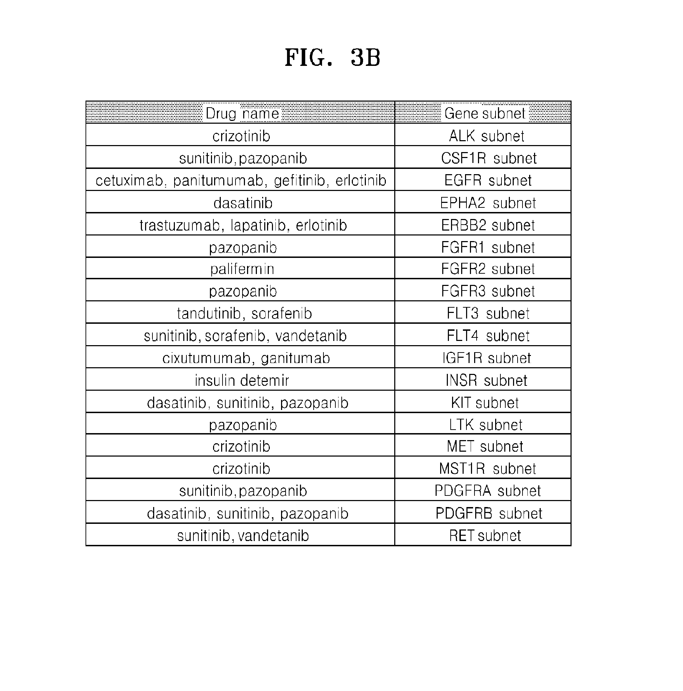

FIG. 3B illustrates a table of subgroups extracted by the subgroup extracting unit 120, according to an embodiment of the present invention. Referring to FIG. 3B, a result in which the drugs described in FIG. 3A are mapped to some subgroups of the gene network is shown. For example, an ALK subnet is mapped to crizotinib because a mechanism of crizotinib corresponds to genes included in the ALK subnet. In addition, a CSFIR subnet is mapped to sunitinib and pazopanib because mechanisms of sunitinib and pazopanib correspond to genes included in the CSFIR subnet. As such, information about subgroups having a gene corresponding to an action of a drug may be based on contents already known in the art. Thus, the subgroup extracting unit 120 extracts subgroups by mapping the subgroups having a gene corresponding to an action of at least one drug to be used based on information already known in the art.

Referring back to FIG. 1, the index generating unit 130 generates at least one index based on gene information included in the subgroups extracted by the subgroup extracting unit 120 to visualize the extracted subgroups.

The at least one index generated by the index generating unit 130 includes indexes for evaluating at least one of a genetic alteration level of each of the extracted subgroups, correlations between the extracted subgroups, and the number of genes included in the extracted subgroups.

An index for evaluating a genetic alteration level of each of the extracted subgroups is estimated by the index generating unit 130 based on genetic alteration levels of genes included in the extracted subgroups.

The index for evaluating a genetic alteration level of each of the extracted subgroups may correspond to an index for indicating the extracted subgroups with different colors according to a genetic alteration level of each of the extracted subgroups.

The genetic alteration level of each of the extracted subgroups may be estimated based on a statistical probability of which genes having a genetic alteration from among the genes included in the individual genome are included in each of the extracted subgroups. This may be estimated by using generally known methods such as the Geneset Analysis, Geneset Enrichment Analysis, and Fisher Exact Test.

For example, the index generating unit 130 may generate an index of a genetic alteration level of each of the extracted subgroups by using Equation 1.

$$p = 1 - \sum_{i=0}^{x-1} \frac{\binom{M}{i}\binom{N-M}{k-i}}{\binom{N}{k}} \qquad (1)$$

In Equation 1, $p$ denotes a probability indicating a genetic alteration level of an extracted subgroup, N denotes the total number of genes in the gene network, k denotes the number of genes having an alteration in a cancer, M denotes the number of genes included in all extracted subgroups, and x denotes the number of genes included in the extracted subgroups from among the genes having an alteration in the cancer.

Equation 1 indicates a value of the probability p of which x or more genes having a genetic alteration are included in the extracted subgroups when k genes having a genetic alteration are selected from among the N genes. Equation 1 is known as the Fisher Exact Test.

However, it will be understood by those of ordinary skill in the art that the index generating unit 130 may estimate the index for evaluating a genetic alteration level of each of the extracted subgroups by using other similar algorithms as described above, such as the Geneset Analysis and Geneset Enrichment Analysis, instead of Equation 1.

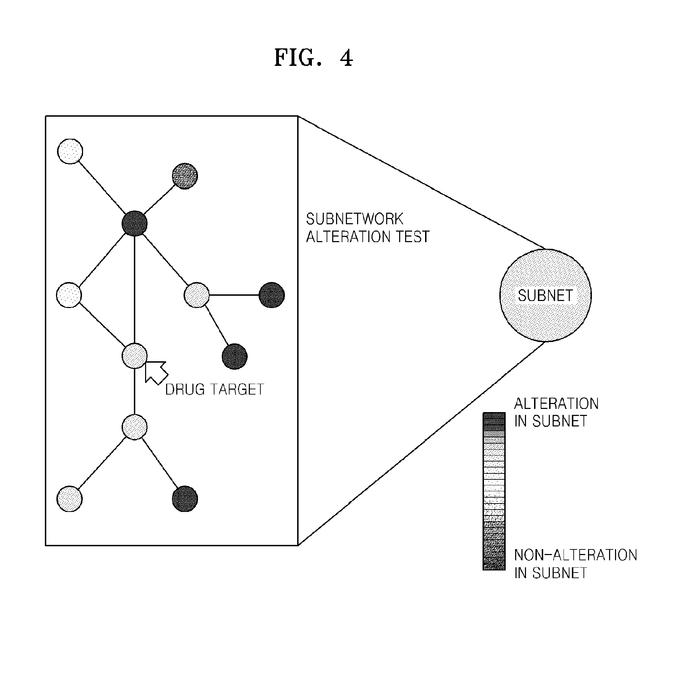

FIG. 4 is a diagram showing an index of a genetic alteration level of an extracted subgroup, which is generated by the index generating unit 130, according to an embodiment of the present invention. Referring to FIG. 4, the genetic alteration level of the extracted subgroup may be represented by using an index indicating a color level.

Referring back to FIG. 1, the index generating unit 130 estimates indexes for evaluating correlations between the extracted subgroups based on distances indicating functionally close levels between genes included in the extracted subgroups. In the current embodiment, the term 'distance' does not mean an actual distance between subgroups but, rather, functional closeness (e.g., degree of relatedness, for instance, in a series of biochemical processes, degree of impact that the expression of one gene has on the function or expression of another, etc.) between genes included in the extracted subgroups.

A distance may be calculated using the number of genes functionally connected to each other between the extracted subgroups. In more detail, a distance may be calculated based on a result obtained by comparing the number of genes functionally connected to each other between the extracted subgroups with the number of genes functionally connected to each other between subgroups randomly sampled from the gene network.

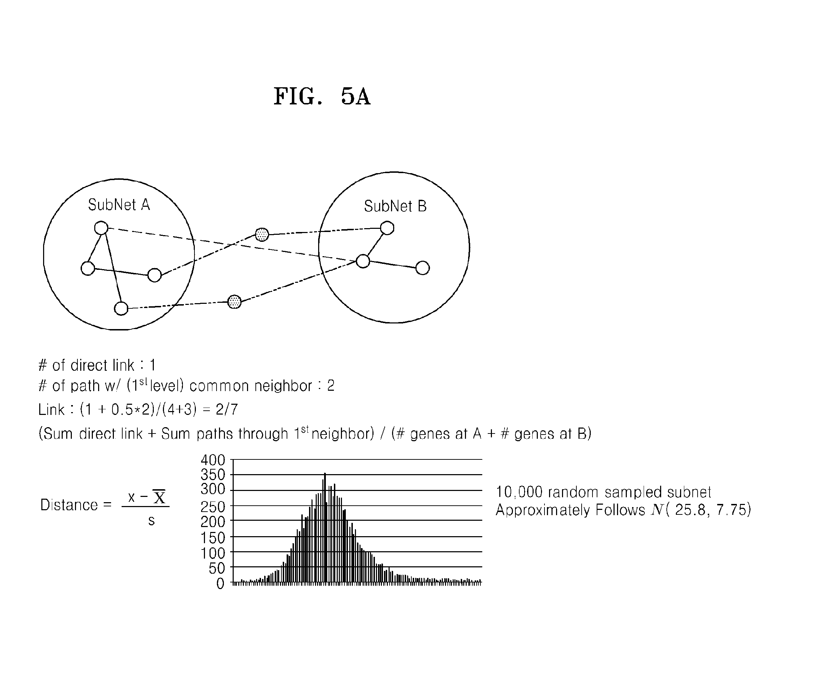

FIG. 5A is a diagram for describing a process of estimating a distance in the index generating unit 130, according to an embodiment of the present invention. When two subgroups are extracted, a correlation between the two subgroups may be estimated.

Referring to FIG. 5A, when two extracted subgroups exist, an inverse number of a distance between the two subgroups is proportional to the number of directly connected genes between the two subgroups and the number of genes connected to each other in the two subgroups by way of a single intervening gene (e.g., an intervening gene not in either subgroup), and is inversely proportional to a sum of the number of genes included in the two subgroups. Here, a weight may be applied to differentiate the importance of the number of directly connected genes from the importance of the number of genes connected to each other by sharing a single gene.

By way of further illustration, the distance between the two subgroups may be estimated using Equation 2.

$$\text{Distance} = \frac{x - \overline{X}}{s} \qquad (2)$$

In Equation 2, x denotes the number of genes connected from a subnet A to a subnet B, $\overline{X}$ denotes the number of genes connected from the subnet A to an arbitrary subnet having the same size as the subnet B, and s denotes a standard deviation of the number of genes connected from the subnet A to the arbitrary subnet having the same size as the subnet B. That is, the distance between the two subgroups may be standardized and estimated by replacing any one subgroup by a subgroup randomly sampled from the gene network.

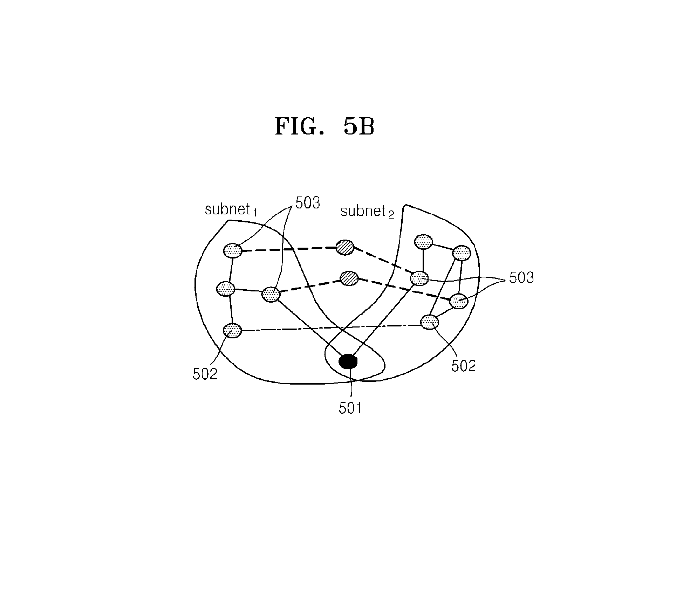

FIG. 5B is a diagram for describing a process of estimating a distance via the index generating unit 130, according to another embodiment of the present invention. When two subgroups are extracted, a correlation between the two subgroups may be estimated.

Referring to FIG. 5B, the index generating unit 130 estimates the distance based on how many gene connection paths exist in comparison with the number of genes included in the two subgroups. In this case, the index generating unit 130 may estimate the distance by using Equation 3.

$$\hat{e}_I = \frac{w_0 \cdot e_0 + w_1 \cdot e_1 + w_2 \cdot e_2}{|V'| + |V''|}$$

(3)

In Equation 3, $\hat{e}_I$ denotes a distance, $|V'|$ denotes the total number of genes included in a subnet 1 of FIG. 5B, $|V''|$ denotes the total number of genes included in a subnet 2 of FIG. 5B, $e_0$ denotes the number of genes commonly included in both the subnet 1 and the subnet 2, $e_1$ denotes the number of paths directly connected between genes remaining by excluding the genes ($e_0$) commonly included in both the subnet 1 and the subnet 2 from among the entire genes included in the subnet 1 and the subnet 2, and $e_2$ denotes the number of paths connecting genes of subnet 1 to genes of subnet 2 with a single intervening gene (e.g., a single intervening gene not included in either subnet 1 or subnet 2). In FIG. 5B, genes corresponding to $e_0$, $e_1$, and $e_2$ are marked by 501, 502, and 503, respectively.

In Equation 3, $w_0$, $w_1$, and $w_2$ denote weights. For example, in a relationship between the genes included in the two subgroups, a weight of two times may be defined for the genes ($e_0$) commonly included in the two subgroups, a weight of one time may be defined for the directly connected genes ($e_1$), and a weight of 0.5 times may be defined for the genes ($e_2$) connected by sharing a single gene. That is, Equation 3 may be used by defining $w_0$=2, $w_1$=1, and $w_2$=0.5. However, it will be understood by those of ordinary skill in the art that the values corresponding to the weights are illustrated for only convenience of description and may be easily modified to meet a using environment.

Referring to FIG. 5B, the index generating unit 130 estimates a distance between the subnet 1 and the subnet 2 as 4/11 by using Equation 3. That is, the index generating unit 130 may estimate distances between the entire extracted subgroups in such a method described above.

Through the illustrations of FIGS. 5A and 5B, a distance estimated between two subgroups may be analyzed to indicate how close the biological functions are between the two subgroups. Thus, it may be determined that the two subgroups are functionally close when the estimated distance is small, whereas the functional similarity between the two subgroups is small when the estimated distance is large. In other words, the distance is inversely proportional to the functional closeness or relatedness of the two subgroups, with a smaller distance indicating a greater degree of closeness and a large distance indicating a lesser degree of closeness. Clinically, when a distance between two subgroups is relatively small, it may be predicted that an interference effect by another subgroup exists when a drug for a certain subgroup is prescribed, i.e., the drug may interact with, or otherwise affect the function of, genes or gene products in both subgroups if the distance between the subgroups is relatively small.

Although estimation of distances is illustrated in the current embodiment as described with reference to FIGS. 5A and 5B, the current embodiment is not limited thereto, and it will be understood by those of ordinary skill in the art that the index generating unit 130 may also generate indexes by using a general method for estimating a correlation between any two groups.

In addition, although only the number of genes connected to each other by sharing a single gene (i.e., genes connected to each other by way of a single intervening gene) existing outside subgroups is used in FIGS. 5A and 5B, a case of sharing more genes may also be used. In particular, in a human gene network, all genes may be actually connected to each other by passing through about 5 steps (i.e., genes connected to each other with about five intervening genes). Thus, it will be understood by those of ordinary skill in the art that a distance may be estimated using genes of the two or more subgroups that are connected to each other with more than one intervening genes (e.g., two or more, three or more, or even four intervening genes), according to another embodiment.

Referring back to FIG. 1, the index generating unit 130 also estimates indexes for evaluating the number of genes included in the extracted subgroups. The indexes for evaluating the numbers of genes included in the extracted subgroups may indicate the relative size of the extracted subgroup based on the number of genes included in the subgroup.

The visualization processor 140 of FIG. 1 processes the extracted subgroups by creating a graphic representation of the extracted subgroups based on the calculated indexes described above, thereby allowing a user to visualize the extracted subgroups. For example, the visualization processor 140 may represent the extracted subgroups by nodes connected to each other.

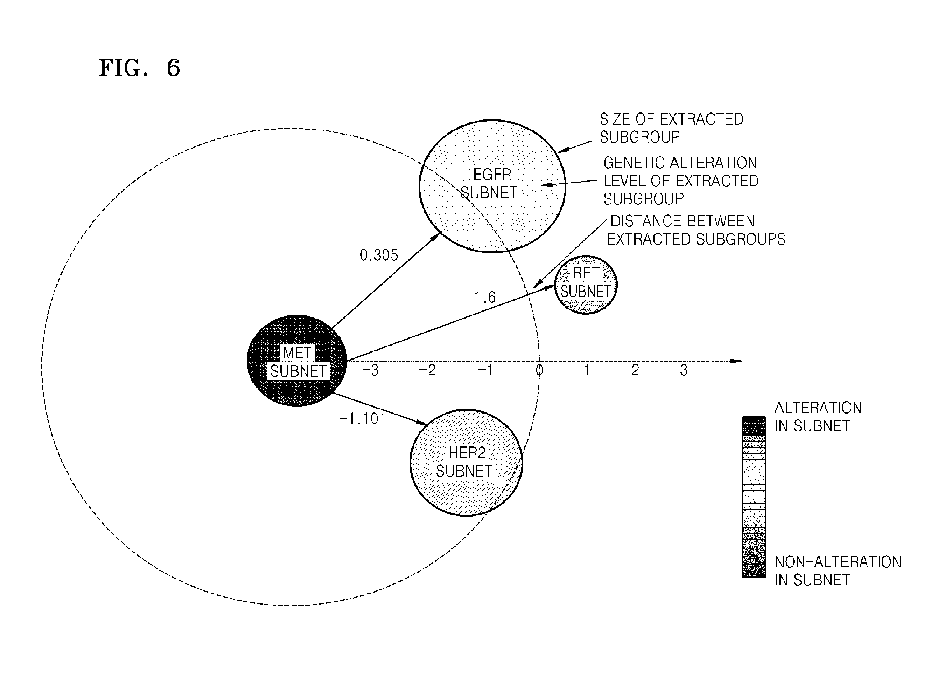

FIG. 6 is a diagram showing a result processed by the visualization processor 140, according to an embodiment of the present invention. Referring to FIG. 6, an MET subnet, an EGFR subnet, an RET subnet, and an HER2 subnet was extracted from a gene network by a subgroup extracting unit 120. The index generating unit 130 generates indexes for the MET subnet, the EGFR subnet, the RET subnet, and the HER2 subnet, and the visualization processor graphically represents the subgroups according to the indexes. For instance, in FIG. 6, the genetic alteration level of each subnet is visualized by a color; the correlation (e.g., distance or relatedness) between subnets is visualized by a numerical distance, allowing the user to differentiate relatedness between subnets from each other according to the numerical distances; and the number of genes included in each subnet is visualized by a size of the shape representing each subnet.

According to another embodiment, the visualization processor 140 may process the visualization in the context of the entire gene network from which the subgroups have been extracted (e.g., FIG 2), whereby only the extracted subgroups on which indexes are reflected in the gene network are highlighted or otherwise visually indicated. The indexes pertaining to the subgroups also may be visually indicated using any suitable technique. For instance, when a user selects a subgroup or node within a subgroup (e.g. places a cursor or mouse pointer on an extracted subgroup or node of the subgroup in a gene network displayed on a screen or display), information about one or more genes included in the extracted subgroups (an alteration of each gene, and so forth) may be visualized.

A result processed by the visualization processor 140 may be output through a user interface unit (not shown), such as a display screen, and provided to a user, such as a therapist.

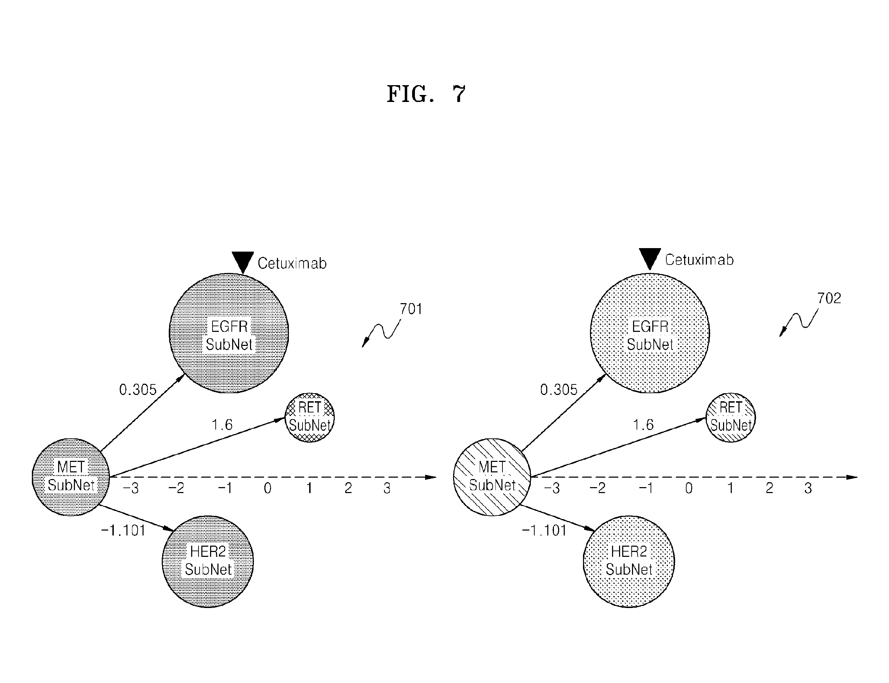

FIG. 7 is a diagram showing visualized results 701 of a colon cancer sample from a responder (cancer responsive to treatment) and visualized results 702 of a colon cancer sample from a non-responder (cancer not responsive to treatment) in relation to Cetuximab, according to an embodiment of the present invention. In the colon cancer sample 701 of the responder, an MET subnet, an EGFR subnet, and an HER2 subnet are displayed with an index indicating a high genetic alteration. That is, the MET subnet, the EGFR subnet, and the HER2 subnet may be marked by, for example, by a color indicating high genetic alteration (e.g. a red-series color). However, in the colon cancer sample 702 of the non-responder, the MET subnet, the EGFR subnet, and the HER2 subnet are displayed with an index indicating a low genetic alteration. That is, the MET subnet, the EGFR subnet, and the HER2 subnet may be marked by, for example, a color indicating low genetic alteration (e.g. green-series color).Accordingly, information indicating whether Cetuximab is effective or not is visually provided to the therapist to perform a therapy with Cetuximab since the MET subnet, the EGFR subnet, and the HER2 subnet that are subgroups of the colon cancer sample 701 of the responder may be provided to a therapist. Similarly, information indicating that it is ineffective even though a therapy is performed with Cetuximab since the MET subnet, the EGFR subnet, and the HER2 subnet that are subgroups of the colon cancer sample 702 of the non-responder may be provided to a therapist.

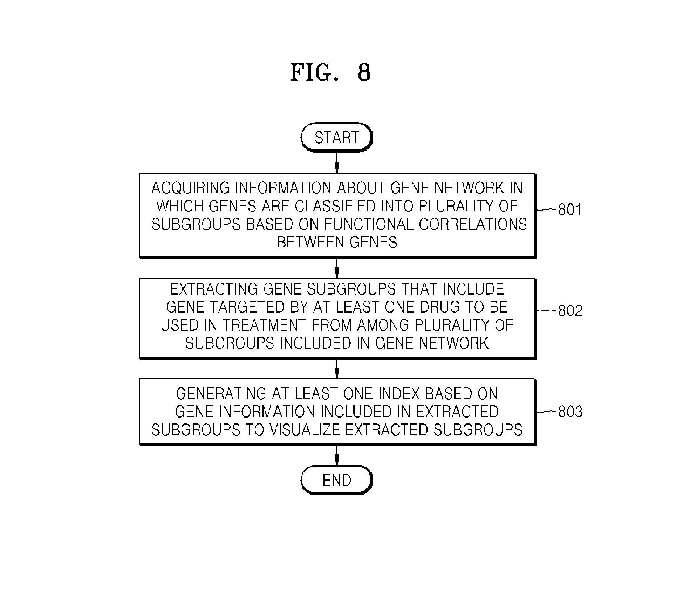

FIG. 8 is a flowchart illustrating a method of analyzing gene information for treatment decision according to an embodiment of the present invention. Referring to FIG. 8, the method consists of operations sequentially processed by the apparatus 10 of FIG. 1. Thus, although omitted in FIG. 8, the contents described with respect to FIG. 1 also apply to the method of FIG. 8.

In operation 801, the data acquisition unit 110 acquires information about a gene network in which genes included in an individual genome are classified into a plurality of subgroups according to functional correlations between the genes.

In operation 802, the subgroup extracting unit 120 extracts subgroups having a gene corresponding to an action of at least one drug to be used from among the plurality of subgroups included in the gene network acquired by the data acquisition unit 110.

In operation 803, the index generating unit 130 generates at least one index based on gene information included in the subgroups extracted by the subgroup extracting unit 120 to visualize the extracted subgroups.

As described above, according to the one or more of the above embodiments of the present invention, information about a gene group causing a disease (e.g., cancer) from among a gene network of a genome of an individual may be visualized with regard to a drug therapy to help a therapist select an effective treatment. In addition, information about gene groups having a genetic alteration, information about correlations between gene groups, and so forth may be provided for an individual patient to help a therapist write an effective prescription. Furthermore, the information may also be used for genetic alteration research, such as development of new medicines, diagnostic markers, and so forth.

The embodiments of the present invention can be written as computer programs and can be implemented in general-use digital computers that execute the programs using a computer-readable recording medium. In addition, a structure of data used in the embodiments of the present invention may be recorded on the computer-readable recording medium through various means. Examples of the computer-readable recording medium include storage media such as magnetic storage media (e.g., ROM, floppy disks, hard disks, etc.) and optical recording media (e.g., CD-ROMs, or DVDs.

In addition, other embodiments of the present invention can also be implemented through computer readable code/instructions in/on a medium, e.g., a computer readable medium, to control at least one processing element to implement any above described embodiment. The medium can correspond to any medium/media permitting the storage and/or transmission of the computer readable code.

The computer readable code can be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more embodiments of the present invention. The media may also be a distributed network, so that the computer readable code is stored/transferred and executed in a distributed fashion. Furthermore, the processing element could include a processor or a computer processor, and processing elements may be distributed and/or included in a single device.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of analyzing gene information for treatment selection, the method comprising:

   acquiring information about a gene network in which genes are classified into a plurality of subgroups based on functional correlations between the genes;
   extracting gene subgroups that include a gene targeted by at least one drug to be used in treatment from among the plurality of subgroups included in the gene network; and
   generating at least one index based on gene information included in the extracted subgroups to visualize the extracted subgroups,
   wherein one or more of the steps of the method are performed using a gene analyzing apparatus.

2. The method of claim 1, wherein the at least one generated index includes an index for evaluating a genetic alteration level of each of the extracted subgroups, evaluating correlations between the extracted subgroups, and/or evaluating the number of genes included in the extracted subgroups.

3. The method of claim 1 or 2, wherein the generating of the at least one index comprises calculating a genetic alteration level of each of the extracted subgroups based on alteration levels of genes included in the extracted subgroups.

4. The method of claim 3, wherein the genetic alteration level of each of the extracted subgroups is calculated based on a statistical probability of which genes having a genetic alteration from among the genes included in a genome are included in each of the extracted subgroups.

5. The method of any one of claims 1 to 4, wherein the generating of the at least one index comprises calculating an index reflecting functional relatedness between genes included in the extracted subgroups.

6. The method of claim 5, wherein the functional relatedness is calculated using the number of genes functionally connected to each other between the extracted subgroups.

7. The method of claim 5, wherein the functional relatedness is calculated based on a result obtained by comparing the number of genes functionally connected to each other between the extracted subgroups with the number of genes functionally connected to each other between subgroups randomly sampled from the gene network.

8. The method of any one of claims 1 to 7, wherein the generating of the at least one index comprises calculating an index reflecting the number of genes included in the extracted subgroups.

9. The method of claim 3 or 8, wherein the at least one generated index is an index indicating each of the extracted subgroups with a different marker according to the number of genes included in the extracted subgroups and/or according to a genetic alteration level of each of the extracted subgroups, preferably the at least one generated index is an index indicating each of the extracted subgroups with a different color according to a generating alteration level of each of the extracted subgroups and/or an index indicating each of the extracted subgroups with a different size according to the number of genes included in the extracted subgroups.

10. The method of any one of claims 1 to 9, further comprising generating a graphic representation of the at least one index applied to the extracted subgroups.

11. A computer readable program for executing the method of any one of claims 1 to 10 as well as a computer readable recording medium storing said computer readable program.

12. An apparatus for analyzing gene information for treatment selection, the apparatus comprising:

a data acquisition unit for acquiring information about a gene network in which genes are classified into a plurality of subgroups based on functional correlations between the genes;
a subgroup extracting unit for extracting gene subgroups that include a gene targeted by at least one drug to be used in treatment from among the plurality of subgroups included in the gene network; and
an index generating unit for generating at least one index based on gene information included in the extracted subgroups to visualize the extracted subgroups.

13. The apparatus of claim 12, wherein the at least one generated index includes an index for evaluating a genetic alteration level of each of the extracted subgroups, evaluating correlations between the extracted subgroups, and/or evaluating the number of genes included in the extracted subgroups.

14. The apparatus of claim 12 or 13, wherein the index generating unit calculates:

- a genetic alteration level of each of the extracted subgroups based on alteration levels of genes included in the extracted subgroups;
- an index reflecting functional relatedness between genes included in the extracted subgroups; and/or
- an index reflecting the number of genes included in the extracted subgroups.

15. The apparatus of any one of claims 12 to 14, further comprising a visualization processor for generating a graphic representation of the at least one index applied to the extracted subgroups.

# FIG. 1

GENE INFORMATION ANALYZING APPARATUS ⟍10

"GENE NETWORK" →

DATA ACQUISITION UNIT ⟍110 →

SUBGROUP EXTRACTING UNIT ⟍120 →

INDEX GENERATING UNIT ⟍130 →

VISUALIZATION PROCESSOR ⟍140 →

DRUG LIST

XXXXXX
XXXXXXXX
XXXXXXX
XXXXXX
XXXXXX

20

# FIG. 2

# FIG. 3A

| Drug List |
| --- |
| crizotinib |
| sunitinib |
| pazopanib |
| cetuximab |
| panitumumab |
| gefitinib |
| erlotinib |
| dasatinib |
| trastuzumab |
| lapatinib |
| palifermin |
| tandutinib |
| sorafenib |
| sunitinib |
| vandetanib |
| cixutumumab |
| ganitumab |
| insulin detemir |

# FIG. 3B

| Drug name | Gene subnet |
|---|---|
| crizotinib | ALK subnet |
| sunitinib, pazopanib | CSF1R subnet |
| cetuximab, panitumumab, gefitinib, erlotinib | EGFR subnet |
| dasatinib | EPHA2 subnet |
| trastuzumab, lapatinib, erlotinib | ERBB2 subnet |
| pazopanib | FGFR1 subnet |
| palifermin | FGFR2 subnet |
| pazopanib | FGFR3 subnet |
| tandutinib, sorafenib | FLT3 subnet |
| sunitinib, sorafenib, vandetanib | FLT4 subnet |
| cixutumumab, ganitumab | IGF1R subnet |
| insulin detemir | INSR subnet |
| dasatinib, sunitinib, pazopanib | KIT subnet |
| pazopanib | LTK subnet |
| crizotinib | MET subnet |
| crizotinib | MST1R subnet |
| sunitinib, pazopanib | PDGFRA subnet |
| dasatinib, sunitinib, pazopanib | PDGFRB subnet |
| sunitinib, vandetanib | RET subnet |

# FIG. 4

SUBNETWORK
ALTERATION TEST

SUBNET

DRUG TARGET

ALTERATION
IN SUBNET

NON-ALTERATION
IN SUBNET

# FIG. 5A

# of direct link : 1

# of path w/ (1st level) common neighbor : 2

Link : $(1 + 0.5*2)/(4+3) = 2/7$

(Sum direct link + Sum paths through 1st neighbor) / (# genes at A + # genes at B)

$$\text{Distance} = \frac{x - \overline{X}}{s}$$

10,000 random sampled subnet
Approximately Follows $N(25.8, 7.75)$

# FIG. 5B

FIG. 6

FIG. 7

# FIG. 8

START

ACQUIRING INFORMATION ABOUT GENE NETWORK IN
WHICH GENES ARE CLASSIFIED INTO PLURALITY OF
SUBGROUPS BASED ON FUNCTIONAL CORRELATIONS
BETWEEN GENES — 801

EXTRACTING GENE SUBGROUPS THAT INCLUDE
GENE TARGETED BY AT LEAST ONE DRUG TO BE
USED IN TREATMENT FROM AMONG PLURALITY OF
SUBGROUPS INCLUDED IN GENE NETWORK — 802

GENERATING AT LEAST ONE INDEX BASED ON
GENE INFORMATION INCLUDED IN EXTRACTED
SUBGROUPS TO VISUALIZE EXTRACTED SUBGROUPS — 803

END